# EUROPEAN PATENT SPECIFICATION

(11) **EP 1 693 027 B1**
(45) Date of publication and mention of the grant of the patent: **02.07.2008**
(21) Application number: 06101562.4
(22) Date of filing: 10.02.2006
(51) Int. Cl.: A61F 9/007

(54) **Phacoemulsification tip**
Spitze zur Phakoemulsifikation
Sonde de phaco-émulsification

(30) Priority: 18.02.2005 US 60827; 21.09.2005 US 232205
(43) Date of publication of application: 23.08.2006
(73) Proprietor: Alcon, Inc., 6331 Hünenberg (CH)
(72) Inventor: Boukhny, Mikhail, Laguna Niguel CA California CA 92677 (US); Chon, James Y., Irvine CA California CA 92604 (US); Dacquay, Bruno, Irvine CA California CA 92688 (US)
(74) Representative: Hanna, Peter William Derek

(56) References cited:
- EP-A- 0 482 847
- US-A- 3 546 498
- US-A- 4 526 571
- US-A- 5 819 571
- US-A- 6 013 046
- US-A1- 2001 027 601

## Description

### Background of the Invention

This invention relates generally to the field of phacoemulsification and more particularly to torsional phacoemulsification cutting tips.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an IOL.

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. Ultrasonic handpieces and cutting tips are more fully described in U.S. Pat. Nos. 3,589,363; 4,223,676; 4, 246, 902; 4,493,694; 4,515,583; 4,589,415; 4,609,368; 4,869,715; 4,922,902; 4,989,583; 5,154,694 and 5,359,996.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

One phacoemulsification tip that has gained widespread acceptance has a belled or flared distal end. Such a tip is described in U.S. Patent No. 4,816,018 (Parisi). Such a design allows for larger lens material purchase as well as increased holding force when vacuum is applied to the tip while maintaining a smaller bore in the shaft of the tip. This combination of features increases anterior chamber stability, by reducing sudden outflow from the anterior chamber when the distal end becomes occluded and this occlusion breaks.

Another phacoemulsification tip is an angled or "bent" tip with or without a flared distal end. These tips are described in U.S. Patent No. 6,039,715 (Mackool), U.S. Patent No. 5,653,724 (Imonti) and U.S. Patent No. 5,154,694 (Kelman). These tips have a predominantly straight shaft with the far distal portion of the shaft being bent on an angle. Bent tips are used by a great many surgeons, and are particularly useful when used in conjunction with a oscillatory phacoemulsification handpiece, such as those described in U.S. Patent No. 6,352,519 (Anis, et al.) and U.S. Patent No. 6,602,193 (Chon) and commercially available as the NeoSoniX^{®} handpiece from Alcon Laboratories, Inc., Fort Worth, Texas. However, some surgeons are reluctant they feel that due to the proximal location of the bend it is more difficult to judge the position of the proximal cutting edge based on the extrapolation of the sleeved portion of the tip.

The state of the art is further represented by US-A-4,526,571 (Wuchinich), US 2001/027601 (Stoddard) and US-A-6,013,046 (Maaskamp) describing angled and bent aspiration tips.

The inventors have discovered that angled phacoemulsification tips are particularly advantageous when used in combination with torsional ultrasound handpiece. Torsional ultrasound handpieces are more fully disclosed in U.S. Patent No. 6,077,285 (Boukhny). Therefore, a need continues to exist for an angled phacoemulsification tip that is safer to use near the posterior capsule.

### Brief Summary of the Invention

The present invention improves upon the prior art by providing a phacoemulsification tip having an arched or curved shaft, in accordance with claims which follow. Such a feature serves to produce more efficient cutting during torsional vibration of the tip while maintaining a greater space between the distal end of the tip and the posterior capsule. The present invention also improves upon the prior art by providing a bent phacoemulsification tip by vibrating the tip torsionally so as to produce a whipping motion in the distal end of the tip.

Accordingly, one objective of the present invention is to provide a phacoemulsification cutting tip having increased efficiency, particularly during torsional ultrasound movement.

Another objective of the present invention is to provide a phacoemulsification cutting tip having a curved shaft.

These and other advantages and objectives of the present invention will become apparent from the detailed description and claims that follow.

### Brief Description of the Drawings

FIG. 1 is a perspective view of the distal end of a typical prior art straight shaft phacoemulsification tip.
FIG. 2 is an elevational view the distal end of a typical prior art angled or bent phacoemulsification tip.
FIG. 3 is an elevational view the distal end of the curved phacoemulsification tip of the present invention.
FIG. 4 is a perspective view of a handpiece and control console that may be used with the present invention.

### Detailed Description of the Invention

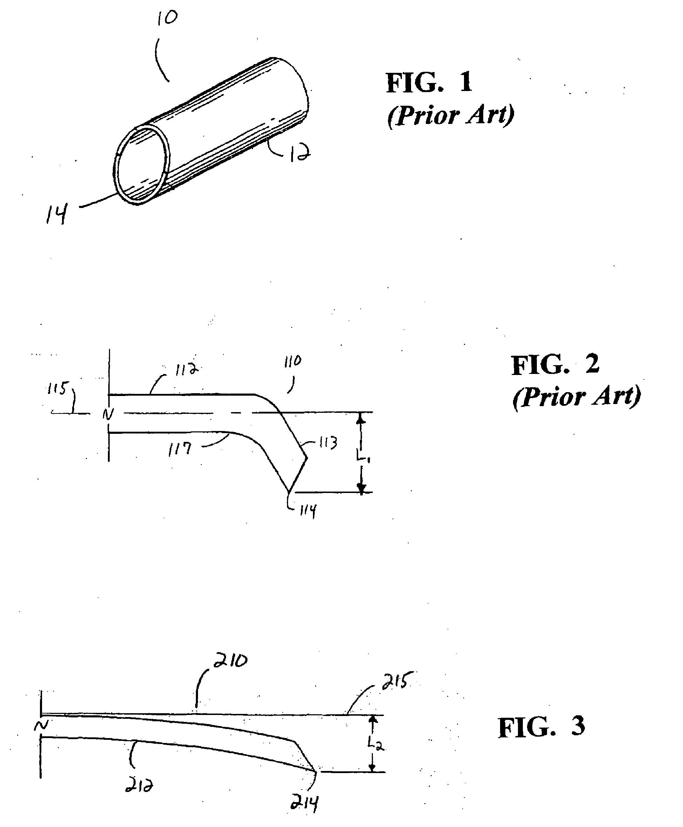

As best seen in FIG. 1, prior art phacoemulsification tip 10 contains shaft 12 that is straight all the way to distal tip 14. As best seen in FIG. 2, prior art phacoemulsification tip 100 contains shaft 112 that is straight up to distal end 113. Distal end 113 is angled or bent on an angle relative to centerline 115 of shaft 112 from intersection 117 of shaft 112 and distal end 113 all the way to distal tip 114.

The inventor has discovered that ultrasonic vibration of tip 110 causes a twisting of shaft 112 that is not present if tip 110 is rotarily oscillated. Such twisting causes distal tip 114 to assume a whipping motion which although less than the rotary motion generated in distal tip 114 when tip 110 is rotarily oscillated, the whipping motion greatly increases the cutting efficiency of tip 110. As discussed above, lateral displacement L₁ of distal tip 114 from longitudinal centerline 115 of shaft 112 can place distal tip 114 near the posterior capsule during surgery, and the exact location of distal tip 114 can be difficult to determine. As a result, some surgeons prefer not to use a phacoemulsification tip of the design shown in FIG. 2.

As best seen in FIG. 3, phacoemulsification tip 210 of the present invention contains shaft 212 that is not straight but instead is bent on a slight arch along the entire length of shaft 112. So constructed, lateral displacement L₂ of distal tip 214 from reference line 215 is less than lateral displacement L₁ of distal tip 114 from centerline 115. Such a construction makes it easier for the surgeon to locate distal tip 214 and maintain a more comfortable distance from the posterior capsule during use, but still benefits from the increase cutting efficiency discussed above.

Cutting tip 210 is preferably made from stainless steel or titanium, but other materials may also be used. Cutting tip 210 preferably has an overall length of between 1.27 cms and 3.81 cms (0.50 inches and 1.50 inches), with 3.05 cms (1.20 inches) being most preferred. Cutting tip 210 may be formed using conventional metalworking technology and preferably is electropolished to remove any burrs.

Shaft 212 is generally tubular, with an outside diameter of between 0.0127 cms and 0.254 cms (0.005 inches and 0.100 inches) and an inside diameter of between 0.002 cms and 0.229 cms (0.001 inches and 0.090 inches). Distal end 214 of shaft 212 may be cut square or cut at any suitable angle between 0° and 90°.

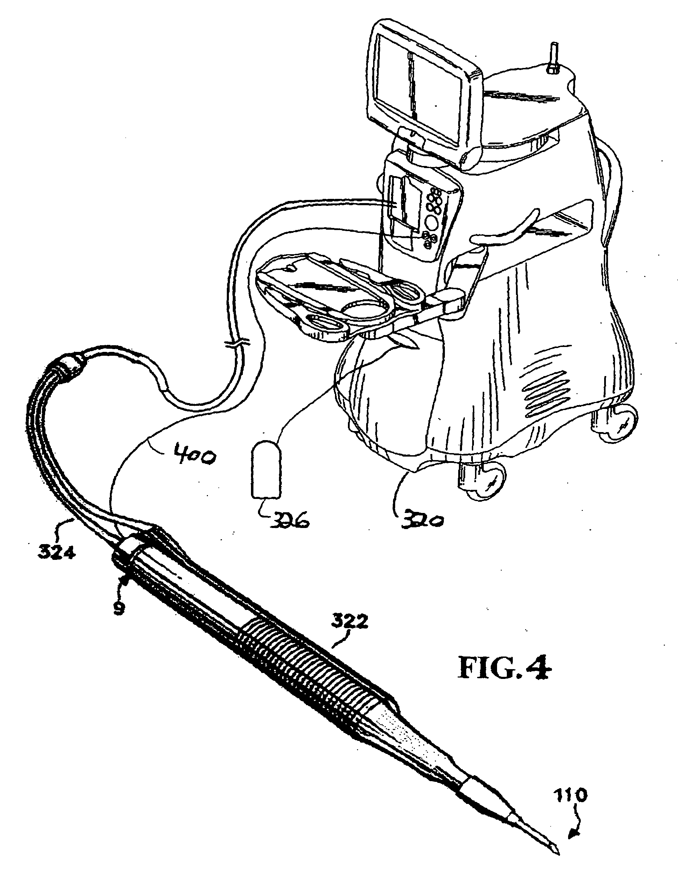

As best seen in FIG. 4, surgical console 320 suitable for use with the present invention may be any commercially available surgical control console such as the INFINITI^{®} surgical systems available from Alcon Laboratories, Inc., Fort Worth, Texas. Console 320 is connected to handpiece 9 through irrigation line 322 and aspiration line 324, and the flow through lines 322 and 324 is controlled by the user, for example, via footswitch 326. Power is supplied to handpiece through electrical cable 400.

This description is given for purposes of illustration and explanation. It will be apparent to those skilled in the relevant art that changes and modifications may be made to the invention described above without departing from its scope.

## Claims

1. A phacoemulsification tip (210), consisting of:
a means for attaching the tip to a surgical handpiece body, and
a tubular shaft (212),
**characterized in that** the shaft is curved relative to a longitudinal centerline (215) along the entire length of the shaft.

2. A phacoemulsification apparatus comprising:
a handpiece,
a surgical console (320) providing an irrigation line (322), an aspiration line (324) and a power supply (400) for connection to the handpiece,
a phacoemulsification tip (210), in accordance with claim 1, attached to the handpiece,
the handpiece (9) comprising means for causing torsional ultrasonic vibration of the tip (110,210), such that the vibration of the tip may be controlled to cause the shaft to twist.

3. The phacoemulsification apparatus of claim 2, wherein the vibration of the tip (110,210) may be further controlled to produce a whipping motion in the distal end (114,214) of the tip.

## Patentansprüche

1. Phakoemulsifikationsspitze (210), die aus Folgendem besteht:
einer Einrichtung zum Anbringen der Spitze an einem Körper eines chirurgischen Handstücks und
einem röhrenförmigen Schaft (212),
**dadurch gekennzeichnet, dass** der Schaft gegenüber einer longitudinalen Mittellinie (215) entlang der gesamten Länge des Schafts gekrümmt ist.

2. Phakoemulsifkationsvorrichtung, die Folgendes aufweist:
ein Handstück,
eine chirurgische Konsole (320), die eine Spülleitung (322), eine Aspirationsleitung (324) und eine Energieversorgung (400) zur Verbindung mit dem Handstück zur Verfügung stellt,
eine Phakoemulsifkationsspitze (210) nach Anspruch 1, die an dem Handstück angebracht ist,
wobei das Handstück (9) eine Einrichtung umfasst, um eine Torsionsschwingung der Spitze (110, 210) hervorzurufen, so dass die Schwingung der Spitze dazu gesteuert werden kann, ein Verdrehen des Schafts hervorzurufen.

3. Phakoemulsifkationsvorrichtung nach Anspruch 2, wobei die Schwingung der Spitze (110, 210) weiterhin dazu gesteuert werden kann, eine peitschende Bewegung in dem distalen Ende (114, 214) der Spitze zu erzeugen.

## Revendications

1. Embout de phacoémulsification (210), comportant :
des moyens pour fixer l'embout sur un corps de pièce à main chirurgicale, et
une tige tubulaire (212),
**caractérisé en ce que** la tige est incurvée par rapport à une ligne centrale longitudinale (215) le long de toute la longueur de l'arbre.

2. Appareil de phacoémulsification comportant :
une pièce à main,
une console chirurgicale (320) fournissant une ligne d'irrigation (322), une ligne d'aspiration (324) et une source de courant (400) destinées à être connectées à la pièce à main,
un embout de phacoémulsification (210), selon la revendication 1, fixé sur la pièce à main,
la pièce à main (9) comportant des moyens pour provoquer une vibration ultrasonore de torsion de l'embout (110, 210), de sorte que la vibration de l'embout puisse être commandée pour amener la tige à se tordre.

3. Appareil de phacoémulsification selon la revendication 2, dans lequel la vibration de l'embout (110, 210) peut en outre être commandée pour produire un mouvement de fouettement de l'extrémité distale (114, 214) de l'embout.
